# FASCICULE DE BREVET EUROPEEN

(11) **EP 4 056 529 B1**
(45) Date de publication et mention de la délivrance du brevet: **03.09.2025**
(21) Numéro de dépôt: 22160730.2
(22) Date de dépôt: 08.03.2022
(51) Int. Cl.: B81C 1/00, A61B 5/00

(54) **PROCÉDÉ DE GRAVURE D'UN TROU ET PROCÉDÉ DE FABRICATION D'UNE MICROAIGUILLE UTILISANT UN TEL TROU**
VERFAHREN ZUM ÄTZEN EINES LOCHS UND VERFAHREN ZUR HERSTELLUNG EINER MIKRONADEL UNTER VERWENDUNG EINES SOLCHEN LOCHS
METHOD FOR ETCHING A HOLE AND METHOD FOR MANUFACTURING A MICRONEEDLE USING SUCH A HOLE

(30) Priorité: 09.03.2021 FR 2102276
(43) Date de publication de la demande: 14.09.2022
(73) Titulaire: WIZP AS, 3183 Horten (NO)
(72) Inventeur: Meunier, Alexandre, 78590 Noisy-le-Roi (FR); ESKIL, Olsen, 3189 HORTEN (NO)
(74) Mandataire: Regimbeau

(56) Documents cités:
- JP-A- 2011 083 387
- HECTOR MALDONADO LOYO ET AL: "Mixed wet and dry etching techniques for microneedles fabrication", ELECTRICAL ENGINEERING COMPUTING SCIENCE AND AUTOMATIC CONTROL (CCE), 2011 8TH INTERNATIONAL CONFERENCE ON, IEEE, 26 October 2011 (2011-10-26), pages 1 - 5, XP032076492, ISBN: 978-1-4577-1011-7, DOI: 10.1109/ICEEE.2011.6106701
- REN LEI ET AL: "A Mini Review of Microneedle Array Electrode for Bio-Signal Recording: A Review", IEEE SENSORS JOURNAL, IEEE, USA, vol. 20, no. 2, 15 January 2020 (2020-01-15), pages 577 - 590, XP011764172, ISSN: 1530-437X, [retrieved on 20191231], DOI: 10.1109/JSEN.2019.2944847

## Description

### DOMAINE DE L'INVENTION

La présente invention concerne un procédé de gravure d'un trou, permettant notamment la fabrication d'une microaiguille d'un capteur adapté pour détecter une grandeur physiologique du corps humain, la microaiguille étant de préférence pleine et avantageusement solide.

### ETAT DE LA TECHNIQUE

Certaines pathologies comme le diabète nécessitent une surveillance quotidienne de paramètres biochimiques du corps humain, en particulier des concentrations en certains composés (la glycémie dans l'exemple du glucose).

Pour cela, il est courant de piquer un point de la peau de sorte à faire perler une goutte de sang, et d'analyser cette goutte soit de façon réactive (par exemple avec une bandelette), soit de façon électronique (par exemple par au moins un capteur analytique), de façon à estimer le ou les paramètres cibles.

On connait aujourd'hui des systèmes évolués bien moins invasifs qui se contentent d'analyser le liquide interstitiel, c'est-à-dire le fluide qui remplit l'espace entre les capillaires sanguins et les cellules. Il a en effet une composition ionique proche de celle du plasma sanguin.

Ces systèmes évolués permettent ainsi de surveiller les paramètres biochimiques souhaités de façon transcutanée, sans nécessité de percer régulièrement la peau et de prélever.

Le document WO 2015/138989 décrit par exemple un capteur de la concentration de glucose dans le corps humain comprenant un réseau de microaiguilles. Chacune de microaiguilles est adaptée à mesurer le glucose par ampérométrie ou par conductimétrie. Une microaiguille peut comprendre un fût de base, monté fixe sur un substrat, et un sommet en pointe monté fixe sur le fût de base. Le sommet en pointe peut comprendre une partie active permettant de détecter le glucose par conductimétrie.

Toutefois, une insertion d'un réseau de microaiguilles dans la peau d'un utilisateur peut être douloureuse si les microaiguilles ne sont pas suffisamment fines. Ainsi, une microaiguille doit présenter un rapport d'aspect élevé (on entend par rapport d'aspect le rapport entre la hauteur et la largeur de la microaiguille), c'est-à-dire supérieur à deux, pour être à la fois assez fine de manière à éviter une douleur de l'utilisateur et à la fois atteindre le liquide interstitiel du derme pour permettre une mesure de l'analyte.

Il est connu d'utiliser un procédé de gravure ionique réactive profonde, appelé également DRIE (acronyme anglo-saxon de *Deep Reactive Ion Etching*) pour fabriquer des éléments présentant un rapport d'aspect élevé.

Toutefois, un tel procédé ne permet que de fabriquer des microaiguilles de révolution, présentant des parois arrondies. Les parois arrondies ne sont pas favorables au dépôt d'un revêtement permettant la détection d'un analyte dans le liquide interstitiel par électrochimie. De plus, un désavantage connu du procédé de gravure ionique réactive profonde est de permettre à des résidus de subsister fixés à l'entrée d'un trou formé par la gravure dans un substrat. De tels résidus peuvent fortement perturber la fabrication des microaiguilles.

Le document HECTOR MALDONADO LOYO ET AL: "Mixed wet and dry etching techniques for microneedles fabrication", ELECTRICAL ENGINEERING COMPUTING SCIENCE AND AUTOMATIC CONTROL (CCE), 2011 8TH INTERNATIONAL CONFERENCE ON, IEEE, 26 octobre 2011 (2011-10-26), pages 1-5, décrit la réalisation de microaiguilles par gravure sèche, puis par gravure humide, dans un substrat de silicium. Un motif de compensation n'est pas décrit.

Les documents JP 2011 083387 A et REN LEI ET AL: "A Mini Review of Microneedle Array Electrode for Bio-Signal Recording: A Review",IEEE SENSORS JOURNAL, IEEE, USA, vol. 20, no. 2, 15 janvier 2020 (2020-01-15), pages 577-590, décrivent également des procédés de fabrication de microaiguilles.

### EXPOSE DE L'INVENTION

Un but de l'invention est de proposer une solution pour graver un trou dans un substrat en silicium, le trou permettant de fabriquer une microaiguille.

Ce but est atteint dans le cadre de la présente invention grâce à un procédé de gravure d'un trou dans un substrat en silicium monocristallin, le substrat comprenant une première face, le trou s'étendant selon une direction principale perpendiculaire à la première face depuis la première face, le trou étant formé par une première partie de la paroi du substrat s'étendant entre la première face du substrat et une première cassure de pente sur une première hauteur selon la direction principale, et par une deuxième partie de la paroi du substrat s'étendant entre la première cassure de pente et un fond du trou sur une deuxième hauteur selon la direction principale, la première cassure de pente formant une concavité du trou, une section de la première partie du trou selon un plan perpendiculaire à la direction principale présentant une première forme prédéterminée, le procédé comprenant les étapes de :
a) fourniture du substrat en silicium monocristallin présentant une première face,
b) dépôt d'un masque de gravure sur la première face, le masque présentant une ouverture définissant une zone de gravure sur la première face, l'ouverture formant un motif de compensation présentant une deuxième forme prédéterminée, la deuxième forme prédéterminée étant différente de la première forme prédéterminée,
c) gravure d'un trou dans le substrat par gravure ionique anisotrope du substrat depuis la zone de gravure, au travers de l'ouverture, à une première profondeur, la première profondeur étant supérieure ou égale à la première hauteur, la gravure anisotrope étant mise en œuvre selon la direction principale,
d) gravure du trou subséquente à la gravure de l'étape c), par gravure humide anisotrope dépendante d'une orientation d'un plan cristallin du substrat, depuis une paroi du substrat formée par la gravure ionique anisotrope lors de l'étape c), la deuxième forme prédéterminée étant configurée pour que, lors de l'étape c) de gravure ionique anisotrope, une section du trou selon un plan perpendiculaire à la direction principale présente la première forme prédéterminée.

L'invention est avantageusement complétée par les caractéristiques suivantes, prises individuellement ou en l'une quelconque de leurs combinaisons techniquement possibles :
- le procédé est un procédé de fabrication d'une microaiguille, la microaiguille comprenant un fût de base et un sommet en pointe agencé sur le fût de base, la microaiguille présentant une deuxième cassure de pente entre le fût de base et le sommet en pointe, la microaiguille s'étendant selon la direction principale entre une base du fut de base et une pointe du sommet en pointe, le fût de base s'étendant selon la direction principale sur une première hauteur depuis la base jusqu'à la deuxième cassure de pente, une section du fût de base selon un plan perpendiculaire à la direction principale présentant la première forme prédéterminée,
- la première forme prédéterminée présente un deuxième axe principal orienté selon une dimension maximale de la première forme prédéterminée, le substrat en silicium monocristallin présentant sur la première face une direction de l'orientation [110] du silicium, le deuxième axe principal formant un angle nul avec la direction de l'orientation [110] du silicium modulo 45°,
- la première forme prédéterminée est rectangulaire ou carrée, et la deuxième forme prédéterminée est choisi parmi une croix, une superposition de plusieurs croix, et une superposition d'un carré d'une croix,
- la microaiguille comprend une partie active de détection recouvrant au moins une partie de la surface du sommet en pointe, la partie active comprenant une face électriquement conductrice adaptée à être recouverte d'un revêtement de détection d'un analyte,
- lequel la gravure mise en œuvre lors de l'étape c) comprend une gravure ionique réactive profonde,
- lequel la gravure mise en œuvre lors de l'étape d) comprend une utilisation d'une solution comprenant au moins un composé choisi parmi de la potasse (KOH) et de l'hydroxyde de tétraméthylammonium (TMAH),
- la première partie de la paroi forme d'un premier angle avec la première face du substrat, une valeur absolue de la moyenne du premier angle étant comprise entre 90° inclus et 125,26° exclus,
- le procédé comprend une étape e), subséquente à l'étape d), de formation d'une couche de matériau électriquement isolant sur la première partie de la paroi et sur la deuxième partie de la paroi,
- le procédé comprend une étape f), subséquente à l'étape e), de remplissage du trou par un matériau électriquement conducteur,
- le procédé comprend une étape g), subséquente à l'étape f), de gravure d'une deuxième face du substrat opposée à la première face de manière à former la microaiguille,
- le procédé comprend une étape h) de gravure partielle de la couche de matériau isolant électriquement,
- la microaiguille présente un rapport d'aspect supérieur à 5, notamment supérieur à 10, et préférentiellement supérieur à 15,
- la première hauteur est supérieure à 500 µm, préférentiellement supérieure à 750 µm.

Un autre aspect de l'invention est une microaiguille obtenue par un procédé selon un mode de réalisation de l'invention.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
[Fig. 1] - la figure 1 illustre schématiquement un procédé selon un mode de réalisation de l'invention.
[Fig. 2] - la figure 2 illustre schématiquement une vue en coupe d'un substrat lors d'une étape d'un procédé selon un mode de réalisation de l'invention.
[Fig. 3] - la figure 3 illustre schématiquement une vue en coupe d'un substrat lors d'une étape d'un procédé selon un mode de réalisation de l'invention.
[Fig. 4] - la figure 4 illustre schématiquement une vue en coupe d'un substrat lors d'une étape d'un procédé selon un mode de réalisation de l'invention.
[Fig. 5] - la figure 5 illustre schématiquement une vue en coupe d'un substrat lors d'une étape d'un procédé selon un mode de réalisation de l'invention.
[Fig. 6] - la figure 6 illustre schématiquement une vue en coupe d'un substrat lors d'une étape d'un procédé selon un mode de réalisation de l'invention.
[Fig. 7] - la figure 7 illustre schématiquement une vue en coupe d'un substrat lors d'une étape d'un procédé selon un mode de réalisation de l'invention.
[Fig. 8] - la figure 8 illustre schématiquement une vue en coupe d'un substrat lors d'une étape d'un procédé selon un mode de réalisation de l'invention.
[Fig. 9] - la figure 9 illustre schématiquement une vue en coupe d'une microaiguille selon un mode de réalisation de l'invention.
[Fig. 10] - la figure 10 illustre un motif de compensation selon un mode de réalisation de l'invention.
[Fig. 11] - la figure 11 illustre schématiquement une gravure anisotrope selon un mode de réalisation de l'invention.
[Fig. 12] - la figure 12 illustre un trou selon un mode de réalisation de l'invention.
[Fig. 13] - la figure 13 illustre une microaiguille selon un mode de réalisation de l'invention.
[Fig. 14] - la figure 14 illustre schématiquement une étape de gravure par voie humide selon un mode de réalisation de l'invention.
[Fig. 15] - la figure 15 illustre schématiquement une étape de gravure par voie humide selon un mode de réalisation de l'invention.
[Fig. 16] - la figure 16 illustre schématiquement une étape de gravure par voie humide selon un mode de réalisation de l'invention.

Sur l'ensemble des figures, les éléments similaires portent des références identiques.

### DESCRIPTION DETAILLEE DE L'INVENTION

### Procédé de fabrication d'un trou 13

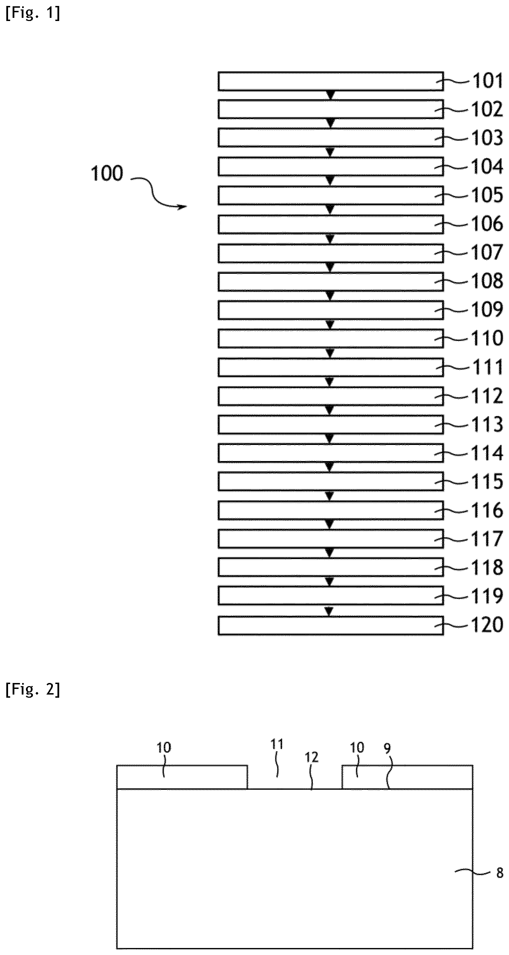

En référence à la figure 1, un aspect de l'invention est un procédé de fabrication, préférentiellement de gravure, d'un trou 13 dans un substrat 8 en silicium monocristallin.

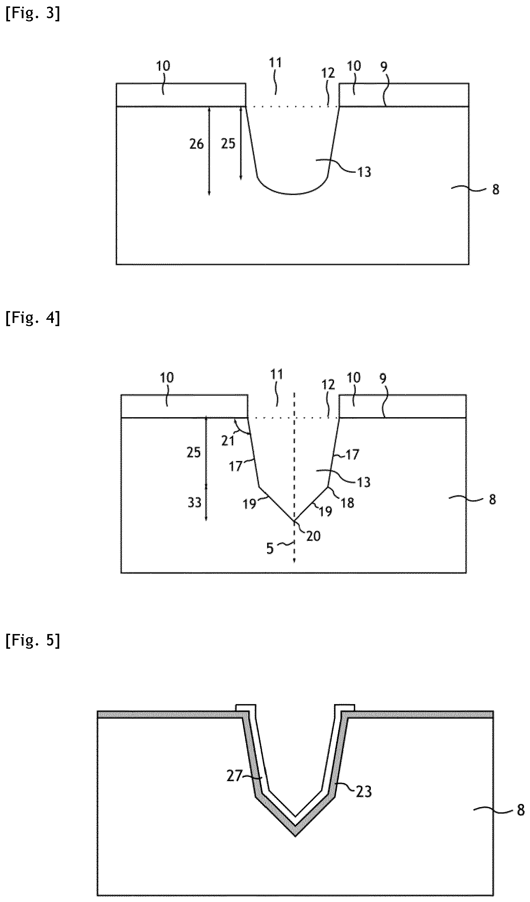

En référence à la figure 4, le substrat 8 comprend une première face 9. Le trou 13 s'étend selon une direction principale 5 perpendiculaire à la première face 9, depuis la première face 9. Le trou 13 est formé par une première partie 17 de la paroi du substrat 8 et par une deuxième partie 19 de la paroi du substrat 8.

La première partie 17 s'étend entre la première face 9 du substrat 8 et une cassure de pente 18, sur une première hauteur 25 selon la direction principale 5. La deuxième partie 19 s'étend entre la cassure de pente 18 et un fond du trou 20, sur une deuxième profondeur 33 selon la direction principale 5. La cassure de pente 18 forme une concavité du trou 13.

Une section de la première partie 17 du trou 13 selon un plan perpendiculaire à la direction principale 5 présente une première forme prédéterminée 30. Préférentiellement, la première forme prédéterminée 30 est rectangulaire ou carrée.

En référence à la figure 1 et à la figure 2, le procédé comprend une étape 101 de fourniture du substrat 8 en silicium monocristallin. Le substrat 8 en silicium monocristallin présent la première face 9.

Le procédé comprend une étape de dépôt d'un masque 10 de gravure sur la première face 9. Le masque 10 présente une ouverture 11 définissant une zone de gravure 12 sur la première face 9. L'ouverture 11 forme un motif de compensation 22 présentant une deuxième forme prédéterminée 31. La deuxième forme prédéterminée 31 est différente de la première forme prédéterminée 30.

Le masque 10 de gravure peut être fabriqué par une étape 102 de dépôt d'une laque photosensible sur la première face 9 puis par une étape 103 de développement de laque photosensible de manière à former l'ouverture 11 selon un motif prédéterminé par l'exposition de laque photosensible.

En référence à la figure 1 et à la figure 3, le procédé comprend une étape 104 de gravure d'un trou 13 dans le substrat 8 par gravure ionique anisotrope du substrat 8 depuis la zone de gravure 12, au travers de l'ouverture 11, à la première profondeur 26. La gravure anisotrope est mise en œuvre selon la direction principale 5.

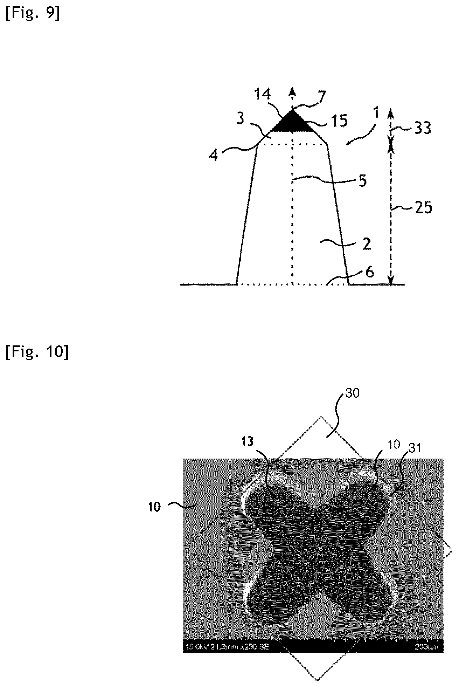

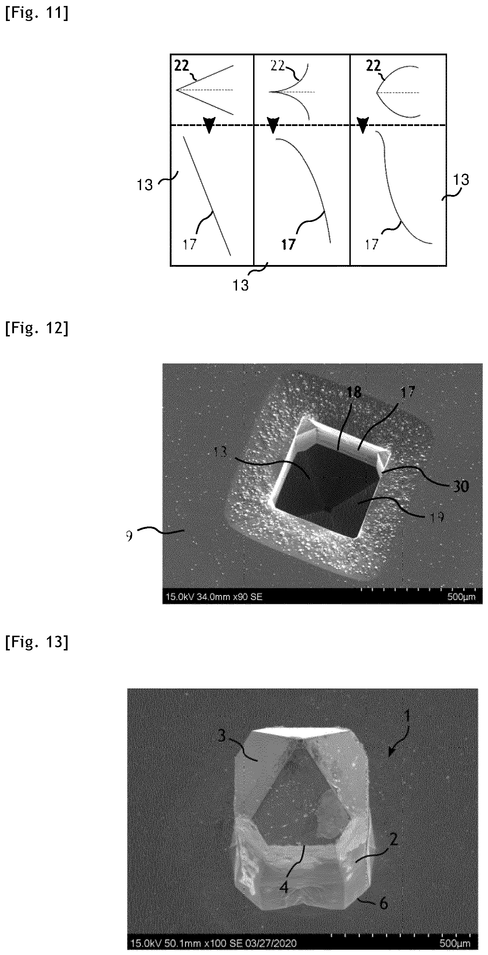

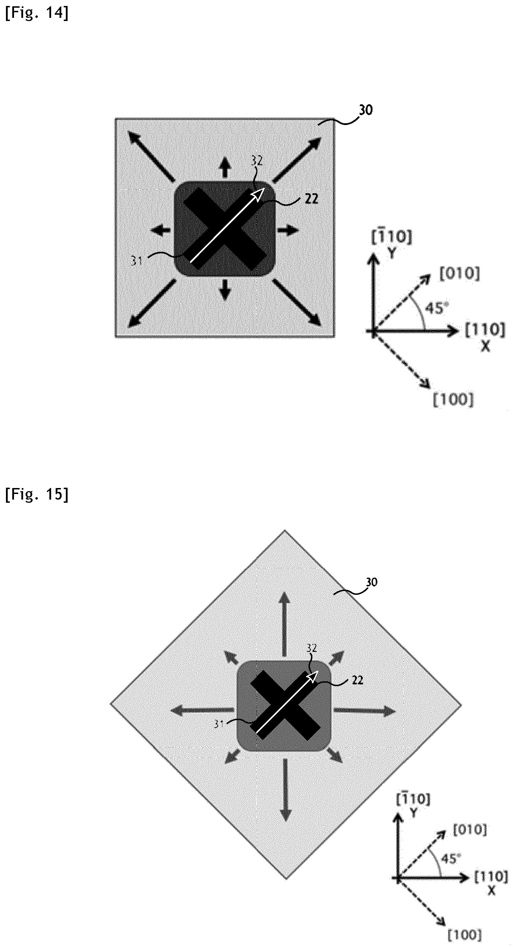

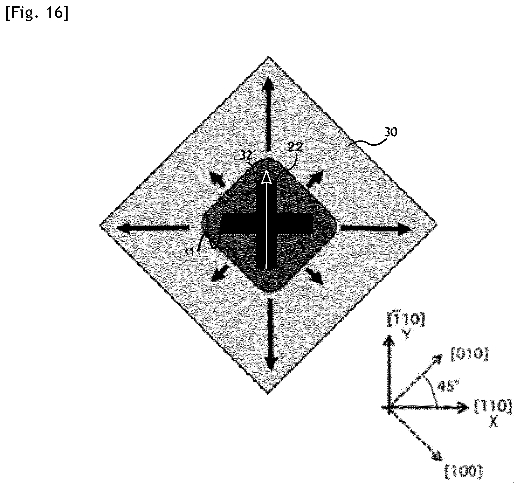

En référence à la figure 10, à la figure 14, à la figure 15, et à la figure 16, la deuxième forme prédéterminée 31 est configurée pour que, lors de l'étape 104 de gravure ionique anisotrope, une section du trou 13 selon un plan perpendiculaire à la direction principale présente la première forme prédéterminée 30. Les inventeurs ont découvert qu'il était possible de contrôler la première forme prédéterminée 30 par la deuxième forme prédéterminée 31 du motif de compensation 22. En référence à la figure 11, la deuxième forme prédéterminée 31 du motif de compensation 22 permet non seulement de contrôler la pente des parois de la première partie 17 du trou 13, mais aussi de contrôler la forme du trou 13 selon une section perpendiculaire à la direction principale 5. Ainsi, il est possible de fabriquer un trou 13 permettant la fabrication d'éléments présentant des parois adaptées au dépôt d'espèces biologiques, notamment d'enzymes adaptées à la détection d'analytes dans le sang.

De préférence, la première forme prédéterminée 30 est rectangulaire ou carrée, et la deuxième forme prédéterminée 31 est choisie parmi une croix, une superposition de plusieurs croix, et une superposition d'un carré d'une croix. La figure 10 illustre un motif de compensation 22 présentant une deuxième forme prédéterminée 31 en croix. Une gravure anisotrope du substrat au travers de ce motif de compensation 22 entraîne la gravure d'un trou 13 dont la section selon un plan perpendiculaire à la direction principale 5 présente une première forme prédéterminée 30 carrée, représenté schématiquement par le carré dans la figure 10. La figure 11 illustre schématiquement, dans les encarts supérieurs, des parties d'une deuxième forme prédéterminée 31 d'un motif de compensation 22, et dans les encarts inférieurs, une section selon un plan parallèle à la direction principale 5 illustrant la pente du trou 13 formé lors de l'étape 104 de gravure anisotrope. La figure 12 est une microphotographie illustrant un trou 13, présentant une première forme prédéterminée 30 carrée, formée avec un motif de compensation 22 présentant une deuxième forme prédéterminée 31 en croix.

Préférentiellement, la gravure mise en œuvre lors de l'étape 104 de gravure anisotrope comprend une gravure ionique réactive profonde (d'acronyme en langue anglaise « D.R.I.E. » pour *Deep Reactive Ion Etching*). Préférentiellement, un procédé de type Bosch peut être utilisé pour mettre en œuvre la gravure anisotrope.

En référence à la figure 4, le motif de compensation 22 permet également de contrôler la pente des parois du trou 13 de la première partie 17. De préférence, la première partie 17 de la paroi forme d'un premier angle 21 avec la première face 9 du substrat 8, une valeur absolue de la moyenne du premier angle 21 étant comprise entre 90° inclus et 125,26° exclus. Ainsi, le trou 13 formé permet la fabrication d'éléments présentant un rapport d'aspect supérieur à 5, notamment supérieur à 10, et préférentiellement supérieur à 15.

Préférentiellement, la première profondeur 26 est supérieure ou égale à 500 µm, notamment supérieure ou égale à 750 µm. La première hauteur 25 est préférentiellement supérieure ou égale à 500 µm, notamment supérieure ou égale à 750 µm. Ainsi, le trou 13 formé permet de fabriquer un élément, notamment une microaiguille, dont la hauteur est adaptée à traverser l'épiderme d'un utilisateur, sans pour autant atteindre les nerfs d'un utilisateur.

Le procédé peut comprendre une étape 105 d'élimination du masque formé dans les étapes 102 et 103. Lorsque le masque est formé par une couche de laque photosensible, l'élimination du masque 10 peut par exemple être mise en œuvre en plongeant le substrat 8 dans un bain d'acétone.

Le procédé comprend une étape 106 de gravure du trou 13 subséquente à l'étape 104, par gravure humide anisotrope dépendante d'une orientation d'un plan cristallin du substrat 8, depuis une paroi du substrat 8 formée par la gravure ionique anisotrope lors de l'étape 104. Ainsi, il est possible de former la deuxième partie 19 du trou 13, de sorte que les parois de la deuxième partie 19 forment une pyramide, présentant quatre faces, s'étendant depuis la cassure de pente 18 jusqu'au fond 20 du trou 13. La deuxième partie 19 du trou présente ainsi des parois planes permettant de fabriquer des éléments adaptés pour le dépôt d'espèces biologiques. La deuxième partie 19 en forme de pyramide est illustrée par la microphotographie en figure 12.

Préférentiellement, la gravure mise en œuvre lors de l'étape 106 comprend une utilisation d'une solution comprenant au moins un composé choisi parmi de la potasse (KOH), de l'hydroxyde de tétraméthylammonium (TMAH), de l'éthylènediamine pyrocatéchol (EDP), de l'Hydrazine anisotropique ou des solutions comportant les molécules suivantes : HF, HFNO3, CH3COOH. Ces composés permettent de graver par voie humide le silicium de manière anisotrope, de manière à former la deuxième partie 19 du trou.

En référence à la figure 14, à la figure 15, et à la figure 16, l'orientation du motif de compensation 22 par rapport aux axes cristallins du substrat 8, permet de choisir l'orientation de la deuxième partie 19 du trou 13. Préférentiellement, la première forme prédéterminée 30 présente un deuxième axe principal 32 orienté selon une dimension maximale de la première forme prédéterminée 30. Le substrat 8 en silicium monocristallin présente sur la première face 9 une direction de l'orientation [110] du silicium. Le deuxième axe principal 32 forme un angle nul avec la direction de l'orientation [110] du silicium modulo 45°.

En référence à la figure 14, le deuxième axe principal 32 forme un angle avec la direction de l'orientation [110] du silicium égal à 45°. Lors d'une gravure humide anisotrope par un composé comprenant du TMAH, la fraction massique en TMAH de la solution de gravure étant inférieure à 10 %, la forme selon une section perpendiculaire à la direction principale 5 de la deuxième partie 19 garde la même orientation, que la forme selon une section perpendiculaire à la direction principale 5 de la première partie 17.

En référence à la figure 15, le deuxième axe principal 32 forme un angle avec la direction de l'orientation 10 du silicium égal à 45°. Lors d'une gravure humide anisotrope par un composé comprenant du TMAH, la fraction massique en TMAH de la solution de gravure étant supérieure à 20 %, préférentiellement égale à 25 %, la forme selon une section perpendiculaire à la direction principale 5 de la deuxième partie 19 présente une orientation pivotée de 45° par rapport à la forme selon une section perpendiculaire à la direction principale 5 de la première partie 17.

En référence à la figure 16, le deuxième axe principal 32 forme un angle avec la direction de l'orientation [110] du silicium égal à 0°. Lors d'une gravure humide anisotrope par un composé comprenant du TMAH, la fraction massique en TMAH de la solution de gravure étant supérieure à 20 %, préférentiellement égale à 25 %, la forme selon une section perpendiculaire à la direction principale 5 de la deuxième partie 19 garde la même orientation, que la forme selon une section perpendiculaire à la direction principale 5 de la première partie 17. Ainsi, la surface gravée lors de l'étape 106 selon ce mode de réalisation présente des parois lisses, adaptées à la fabrication d'éléments comprenant des parois adaptées au dépôt d'espèces biologiques.

### Fabrication d'une microaiguille 1

Un autre aspect de l'invention est un procédé de fabrication d'une microaiguille 1. Le procédé de fabrication d'une microaiguille 1 comprend les étapes de gravure d'un trou 13. Le trou 13 permet de fabriquer la microaiguille 1 dans le même substrat 8. En référence à la figure 9, la microaiguille 1 comprenant un fût de base 2 et un sommet en pointe 3 agencé sur le fût de base 2. La microaiguille 1 présente une cassure de pente 4 entre le fût de base 2 et le sommet en pointe 3. La microaiguille 1 s'étend selon la direction principale 5 entre une base 6 du fut de base 2 et une pointe 7 du sommet 3 en pointe. Le fût de base 2 s'étend selon la direction principale 5 sur la première hauteur 25 depuis la base 6 jusqu'à la cassure de pente 4. Une section du fût de base 2 selon un plan perpendiculaire à la direction principale 5 présente la première forme prédéterminée 30.

Préférentiellement, la microaiguille 1 comprend une partie active 14 de détection recouvrant au moins une partie de la surface du sommet en pointe 3. La partie active 14 comprenant une face électriquement conductrice 15 adaptée à être recouverte d'un revêtement de détection d'un analyte. Ainsi, il est possible de contrôler un courant électrique/une tension électrique sur la partie active 14 de la microaiguille, de manière à détecter un analyte de l'utilisateur par électrochimie.

En référence à la figure 5, le procédé de fabrication de la microaiguille 1 peut comprendre une étape 107 de formation d'une couche de matériau électriquement isolant 23, préférentiellement d'oxyde thermique, sur les parois du substrat 8. Ainsi, les parois de la première partie 17 et de la deuxième partie 19 du trou 13 sont isolées électriquement.

Le procédé de fabrication de la microaiguille 1 peut comprendre au moins une étape, subséquente à l'étape 107, de formation d'une couche de matériau électriquement conducteur 27 sur la couche de matériau électriquement isolant 23 déposée précédemment. Préférentiellement, le procédé comprend une étape 108 de dépôt d'une couche de chrome sur la couche de matériau électriquement isolant 23, suivi d'une étape 109 de dépôt d'une couche d'or sur la couche de chrome déposait lors de l'étape 108. Ces couches de matériau électriquement conducteur permettent à la fois de former la partie active 14 de la microaiguille, et d'assurer l'adhérence entre un matériau remplissant ultérieurement le trou 13 et les parois du trou 13.

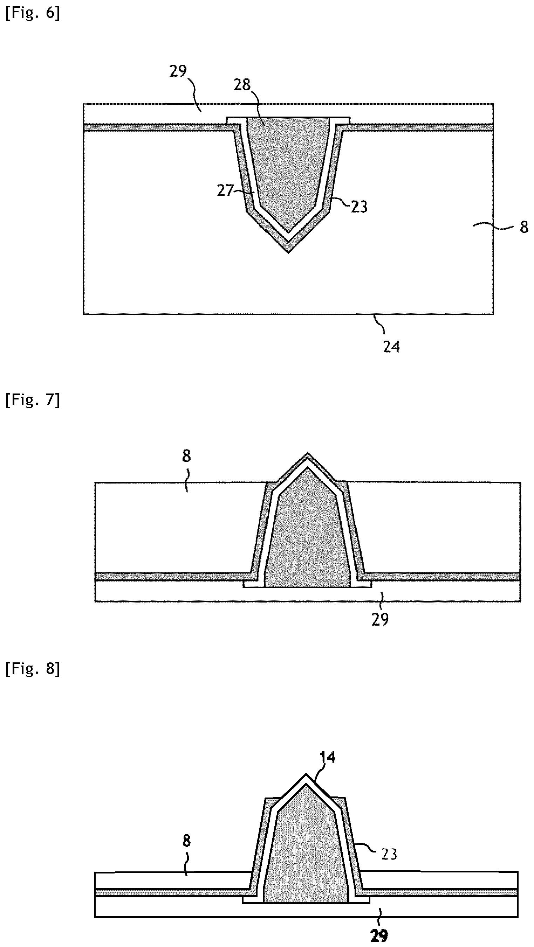

En référence à la figure 6, le procédé de fabrication de la microaiguille 1 peut comprendre une étape, subséquente à l'étape de formation d'une couche de matériau électriquement isolant 23, de remplissage du trou 13 par un matériau électriquement conducteur 28. L'étape de remplissage du trou 13 par un matériau électriquement conducteur peut être une étape 110 de remplissage du trou 13 par du cuivre par une méthode d'éléctroplacage (ou *electroplating* en anglais).

Le procédé peut comprendre une étape 111, ultérieur à l'étape 110, d'abrasion du cuivre, déposé lors de l'étape 110, en dehors du trou 13. L'étape 111 peut être mise en œuvre de manière à laisser une couche de cuivre sur la première face du substrat 8 en dehors du trou 13.

La première face 9 du substrat 8 peut préférentiellement être recouverte d'une couche protectrice 29, préférentiellement une couche de nitrure, permettant de protéger la première face 9 des attaques chimiques lors des étapes ultérieures du procédé de fabrication de la microaiguille.

Le procédé peut comprendre une étape 112 de gravure d'une couche d'oxyde thermique formée sur une deuxième face 24 du substrat 8, la deuxième face 24 étant opposée à la première face 9 du substrat 8.

En référence à la figure 7, le procédé peut comprendre une étape, subséquente à l'étape de remplissage du trou, de gravure de la deuxième face 24 du substrat 8 opposée à la première face 9 de manière à former ou révéler la microaiguille 1. De préférence, cette étape peut comprendre une étape 113 de gravure humide du silicium du substrat 8, depuis la deuxième face 24. Préférentiellement, la gravure humide du silicium est arrêtée lorsque la deuxième face 24 du substrat a déjà atteint la deuxième partie 19, mais n'a pas encore atteint la première partie 17. Préférentiellement, la gravure humide du silicium est arrêtée lorsque la deuxième face 24 du substrat 8 atteint la cassure de pente 18.

En référence à la figure 7, le procédé peut comprendre une étape 114 de gravure partielle de la couche 23 de matériau isolant électriquement. De préférence, la gravure partielle de la couche 23 de matériau électriquement isolant est arrêtée de sorte que l'épaisseur de la couche 23 de matériau électriquement isolant soit supérieure à 125 nm.

En référence à la figure 8, le procédé peut comprendre une étape 115, ultérieure à l'étape 114, de gravure humide du silicium du substrat 8, depuis la deuxième face 24. Cette étape permet de former le fût de base 2 de la microaiguille. De préférence, la gravure humide de l'étape 115 est arrêtée avant d'avoir gravé la totalité du silicium substrat 8. La gravure du silicium lors de l'étape 115 n'est pas parfaitement sélective au regard de la gravure de la couche 23 de matériau isolant électriquement restante lors de l'étape 114. Ainsi, lors de l'étape 115, la couche 23 restante lors de l'étape 114 est elle aussi gravée, de sorte que le sommet en pointe 3 de la microaiguille est formé par la couche 27 de matériau électriquement conducteur.

Le procédé de fabrication de la microaiguille 1 peut également comprendre une étape 116 de préparation de la galette sur laquelle est fabriquée la microaiguille en la nettoyant avec une solution de persulfate de sodium, une étape 117 de gravure des couches de chrome et d'or sur le sommet en pointe 3, une étape 118 de dépôt de nickel sélective sur la couche de cuivre sur le sommet en pointe 3, une étape 119 de dépôt d'or sélective sur la couche de nickel sur le sommet en pointe 3, une étape 120 de déprotection par suppression du masque en nitrure, et une étape 121 de découpe de la galette de manière à séparer les différentes microaiguilles 1 formée sur la même galette.

## Revendications

1. Procédé de gravure d'un trou (13) dans un substrat (8) en silicium monocristallin, le substrat (8) comprenant une première face (9), le trou (13) s'étendant selon une direction principale (5) perpendiculaire à la première face (9), depuis la première face (9), le trou (13) étant formé par une première partie (17) de la paroi du substrat (8) s'étendant entre la première face (9) du substrat (8) et une cassure de pente (18) sur une première hauteur (25) selon la direction principale (5), et par une deuxième partie (19) de la paroi du substrat s'étendant entre la cassure de pente (18) et un fond (20) du trou sur une deuxième hauteur (33) selon la direction principale (5), la cassure de pente (18) formant une concavité du trou (13), une section de la première partie (17) du trou (13) selon un plan perpendiculaire à la direction principale (5) présentant une première forme prédéterminée (30), le procédé comprenant les étapes de :
a) fourniture du substrat (8) en silicium monocristallin présentant une première face (9),
b) dépôt d'un masque (10) de gravure sur la première face (9), le masque (10) présentant une ouverture (11) définissant une zone de gravure (12) sur la première face (9), l'ouverture (11) formant un motif de compensation (22) présentant une deuxième forme prédéterminée (31), la deuxième forme prédéterminée (31) étant différente de la première forme prédéterminée (30),
c) gravure d'un trou (13) dans le substrat (8) par gravure ionique anisotrope du substrat (8) depuis la zone de gravure (12), au travers de l'ouverture (11), à une première profondeur (26), la première profondeur (26) étant supérieure ou égale à la première hauteur (25), la gravure anisotrope étant mise en œuvre selon la direction principale (5),
d) gravure du trou (13) subséquente à la gravure de l'étape c), par gravure humide anisotrope dépendante d'une orientation d'un plan cristallin du substrat (8), depuis une paroi du substrat (8) formée par la gravure ionique anisotrope lors de l'étape c), la deuxième forme prédéterminée (31) étant configurée pour que, lors de l'étape c) de gravure ionique anisotrope, une section du trou (13) selon un plan perpendiculaire à la direction principale présente la première forme prédéterminée (30).

2. Procédé selon la revendication précédente, le procédé étant un procédé de fabrication d'une microaiguille (1), la microaiguille (1) comprenant un fût de base (2) et un sommet en pointe (3) agencé sur le fût de base (2), la microaiguille (1) présentant une cassure de pente (4) entre le fût de base (2) et le sommet en pointe (3), la microaiguille (1) s'étendant selon la direction principale (5) entre une base (6) du fut de base (2) et une pointe (7) du sommet (3) en pointe, le fût de base (2) s'étendant selon la direction principale (5) sur la première hauteur (25) depuis la base (6) jusqu'à la cassure de pente (4), une section du fût de base (2) selon un plan perpendiculaire à la direction principale (5) présentant la première forme prédéterminée (30).

3. Procédé selon la revendication précédente, dans lequel la microaiguille (1) comprend une partie active (14) de détection recouvrant au moins une partie de la surface du sommet en pointe (3), la partie active (14) comprenant une face électriquement conductrice (15) adaptée à être recouverte d'un revêtement de détection d'un analyte.

4. Procédé selon l'une des revendications précédentes, dans lequel la première forme prédéterminée (30) présente un deuxième axe principal (32) orienté selon une dimension maximale de la première forme prédéterminée (30), le substrat (8) en silicium monocristallin présentant sur la première face (9) une direction de l'orientation [110] du silicium, le deuxième axe principal (32) formant un angle nul avec la direction de l'orientation [110] du silicium modulo 45°.

5. Procédé selon l'une des revendications précédentes, dans lequel la première forme prédéterminée (30) est rectangulaire ou carrée, et dans lequel la deuxième forme prédéterminée (31) est choisi parmi une croix, une superposition de plusieurs croix, et une superposition d'un carré d'une croix.

6. Procédé selon l'une des revendications précédentes, dans lequel la gravure mise en œuvre lors de l'étape c) comprend une gravure ionique réactive profonde.

7. Procédé selon l'une des revendications précédentes, dans lequel la gravure mise en œuvre lors de l'étape d) comprend une utilisation d'une solution comprenant au moins un composé choisi parmi de la potasse (KOH) et de l'hydroxyde de tétraméthylammonium (TMAH).

8. Procédé selon l'une des revendications précédentes, dans lequel la première partie (17) de la paroi forme d'un premier angle (21) avec la première face (9) du substrat (8), une valeur absolue de la moyenne du premier angle (21) étant comprise entre 90° inclus et 125,26° exclus.

9. Procédé selon l'une des revendications 2 à 8, comprenant une étape e), subséquente à l'étape d), de formation d'une couche (23) de matériau électriquement isolant sur la première partie (17) de la paroi et sur la deuxième partie (19) de la paroi.

10. Procédé selon la revendication précédente, comprenant une étape f), subséquente à l'étape e), de remplissage du trou (13) par un matériau électriquement conducteur.

11. Procédé selon la revendication précédente, comprenant une étape g), subséquente à l'étape f), de gravure d'une deuxième face (24) du substrat (8) opposée à la première face (9) de manière à former ou révéler la microaiguille (1).

12. Procédé selon la revendication précédente, comprenant une étape h) de gravure partielle de la couche (23) de matériau isolant électriquement.

13. Procédé selon l'une des revendications précédentes, dans lequel la microaiguille (1) présente un rapport d'aspect supérieur à 5, notamment supérieur à 10, et préférentiellement supérieur à 15.

14. Procédé selon l'une des revendications précédentes, dans lequel la première hauteur (25) est supérieure à 500 µm, préférentiellement supérieure à 750 µm.

## Patentansprüche

1. Verfahren zum Ätzen eines Lochs (13) in ein Substrat (8) aus monokristallinem Silizium, wobei das Substrat (8) eine erste Fläche (9) aufweist, wobei sich das Loch (13) gemäß einer Hauptrichtung (5) senkrecht zur ersten Fläche (9) von der ersten Fläche (9) aus erstreckt, wobei das Loch (13) von einem ersten Teil (17) der Wand des Substrats (8) gebildet wird, der sich zwischen der ersten Fläche (9) des Substrats (8) und einem Neigungsbruch (18) auf einer ersten Höhe (25) gemäß der Hauptrichtung (5) erstreckt, und von einem zweiten Teil (19) der Wand des Substrats, der sich zwischen dem Neigungsbruch (18) und einem Boden (20) des Lochs auf einer zweiten Höhe (33) gemäß der Hauptrichtung (5) erstreckt, wobei der Neigungsbruch (18) eine Konkavität des Lochs (13) bildet,
wobei ein Abschnitt des ersten Teils (17) des Lochs (13) gemäß einer zur Hauptrichtung (5) senkrechten Ebene eine erste vorbestimmte Form (30) aufweist,
wobei das Verfahren die folgenden Schritte umfasst:
a) Bereitstellen des Substrats (8) aus monokristallinem Silizium, das eine erste Fläche (9) aufweist,
b) Aufbringen einer Ätzmaske (10) auf die erste Fläche (9), wobei die Maske (10) eine Öffnung (11) aufweist, die einen Ätzbereich (12) auf der ersten Fläche (9) definiert, wobei die Öffnung (11) ein Ausgleichsmuster (22) bildet, das eine zweite vorbestimmte Form (31) aufweist, wobei die zweite vorbestimmte Form (31) von der ersten vorbestimmten Form (30) verschieden ist,
c) Ätzen eines Lochs (13) in das Substrat (8) durch anisotropes Ionenätzen des Substrats (8) ab dem Ätzbereich (12) durch die Öffnung (11) hindurch in einer ersten Tiefe (26), wobei die erste Tiefe (26) größer oder gleich der ersten Höhe (25) ist, wobei das anisotrope Ätzen gemäß der Hauptrichtung (5) erfolgt,
d) Ätzen des Lochs (13) unmittelbar im Anschluss an das Ätzen von Schritt c) durch anisotropes Nassätzen in Abhängigkeit von einer Ausrichtung einer Kristallebene des Substrats (8) ab einer durch das anisotrope Ionenätzen in Schritt c) gebildeten Wand des Substrats (8),
wobei die zweite vorbestimmte Form (31) ausgelegt ist, damit in Schritt c) des anisotropen Ionenätzens ein Abschnitt des Lochs (13) gemäß einer zur Hauptrichtung senkrechten Ebene die erste vorbestimmte Form (30) aufweist.

2. Verfahren nach vorhergehendem Anspruch, wobei das Verfahren ein Verfahren zur Herstellung einer Mikronadel (1) ist, wobei die Mikronadel (1) einen Basisschaft (2) und eine auf dem Basisschaft (2) eingerichtete spitze Spitze (3) umfasst, wobei die Mikronadel (1) einen Neigungsbruch (4) zwischen dem Basisschaft (2) und der spitzen Spitze (3) aufweist, wobei sich die Mikronadel (1) gemäß der Hauptrichtung (5) zwischen einer Basis (6) des Basisschafts (2) und einer Spitze (7) der spitzen Spitze (3) erstreckt, wobei sich der Basisschaft (2) gemäß der Hauptrichtung (5) auf der ersten Höhe (25) von der Basis (6) bis zum Neigungsbruch (4) erstreckt, wobei ein Abschnitt des Basisschafts (2) gemäß einer zur Hauptrichtung (5) senkrechten Ebene die erste vorbestimmte Form (30) aufweist.

3. Verfahren nach vorhergehendem Anspruch, wobei die Mikronadel (1) einen aktiven Detektionsteil (14) umfasst, der mindestens einen Teil der Oberfläche der spitzen Spitze (3) bedeckt, wobei der aktive Teil (14) eine elektrisch leitende Fläche (15) umfasst, die geeignet ist, mit einer Beschichtung zur Detektion eines Analyten bedeckt zu sein.

4. Verfahren nach einem der vorstehenden Ansprüche, wobei die erste vorbestimmte Form (30) eine zweite Hauptachse (32) aufweist, die gemäß einer maximalen Abmessung der ersten vorbestimmten Form (30) ausgerichtet ist, wobei das Substrat (8) aus monokristallinem Silizium auf der ersten Fläche (9) eine Richtung der Ausrichtung [110] des Siliziums aufweist, wobei die zweite Hauptachse (32) einen Winkel von null mit der Richtung der Ausrichtung [110] des Siliziums modulo 45° bildet.

5. Verfahren nach einem der vorstehenden Ansprüche, wobei die erste vorbestimmte Form (30) rechteckig oder quadratisch ist und wobei die zweite vorbestimmte Form (31) aus einem Kreuz, einer Überlagerung mehrerer Kreuze und einer Überlagerung eines Quadrats mit einem Kreuz ausgewählt ist.

6. Verfahren nach einem der vorstehenden Ansprüche, wobei das in Schritt c) durchgeführte Ätzen ein tiefes reaktives Ionenätzen umfasst.

7. Verfahren nach einem der vorstehenden Ansprüche, wobei das in Schritt d) durchgeführte Ätzen eine Verwendung einer Lösung umfasst, die mindestens eine Verbindung umfasst, die aus Kaliumhydroxid (KOH) und Tetramethylammoniumhydroxid (TMAH) ausgewählt ist.

8. Verfahren nach einem der vorstehenden Ansprüche, wobei der erste Teil (17) der Wand einen ersten Winkel (21) mit der ersten Fläche (9) des Substrats (8) bildet, wobei ein Absolutwert des Mittelwerts des ersten Winkels (21) zwischen 90° einschließlich und 125,26° ausschließlich liegt.

9. Verfahren nach einem der Ansprüche 2 bis 8, umfassend einen Schritt e) unmittelbar nach Schritt d) des Bildens einer Schicht (23) aus elektrisch isolierendem Material auf dem ersten Teil (17) der Wand und auf dem zweiten Teil (19) der Wand.

10. Verfahren nach vorhergehendem Anspruch, umfassend einen Schritt f) unmittelbar nach Schritt e) des Füllens des Lochs (13) mit einem elektrisch leitenden Material.

11. Verfahren nach vorhergehendem Anspruch, umfassend einen Schritt g) unmittelbar nach Schritt f) des Ätzens einer zweiten Fläche (24) des Substrats (8) gegenüber der ersten Fläche (9), um die Mikronadel (1) zu bilden oder freizulegen.

12. Verfahren nach vorhergehendem Anspruch, umfassend einen Schritt h) des teilweisen Ätzens der Schicht (23) aus elektrisch isolierendem Material.

13. Verfahren nach einem der vorhergehenden Ansprüche, wobei die Mikronadel (1) ein Seitenverhältnis von über 5, insbesondere über 10 und vorzugsweise über 15 aufweist.

14. Verfahren nach einem der vorstehenden Ansprüche, wobei die erste Höhe (25) größer als 500 µm, vorzugsweise größer als 750 µm ist.

## Claims

1. A method of etching a hole (13) in a substrate (8) of single crystal silicon, the substrate (8) comprising a first face (9), the hole (13) extending in a main direction (5) perpendicular to the first face (9) from the first face (9), the hole (13) being formed by a first portion (17) of the wall of the substrate (8) extending between the first face (9) of the substrate (8) and a slope break (18) over a first height (25) in the main direction (5), and by a second portion (19) of the wall of the substrate extending between the slope break (18) and a bottom (20) of the hole over a second height (33) in the main direction (5), the slope break (18) forming a concavity of the hole (13),
a section of the first portion (17) of the hole (13) in a plane perpendicular to the main direction (5) having a first predetermined shape (30),
the method comprising the steps of:
a) providing the substrate (8) of monocrystalline silicon having a first face (9),
b) depositing an etching mask (10) on the first face (9), the mask (10) having an opening (11) defining an etching zone (12) on the first face (9), the opening (11) forming a compensation pattern (22) having a second predetermined shape (31), the second predetermined shape (31) being different from the first predetermined shape (30),
c) etching a hole (13) in the substrate (8) by anisotropic ion etching of the substrate (8) from the etch zone (12), through the opening (11), to a first depth (26), the first depth (26) being greater than or equal to the first height (25), the anisotropic etching being carried out along the main direction (5),
d) etching the hole (13) subsequent to the etching in step c), by anisotropic wet etching dependent on an orientation of a crystal plane of the substrate (8), from a wall of the substrate (8) formed by the anisotropic ion etching in step c),
the second predetermined shape (31) being configured so that, during step c) of anisotropic ion etching, a section of the hole (13) along a plane perpendicular to the main direction has the first predetermined shape (30).

2. Method according to the preceding claim, the method being a method of manufacturing a microneedle (1), the microneedle (1) comprising a base shank (2) and a pointed apex (3) arranged on the base shank (2), the microneedle (1) having a slope break (4) between the base shank (2) and the pointed apex (3), **characterized in that** the microneedle (1) extending in the main direction (5) between a base (6) of the base shaft (2) and a tip (7) of the pointed apex (3), the base shaft (2) extending in the main direction (5) over the first height (25) from the base (6) to the slope break (4), a section of the base shaft (2) in a plane perpendicular to the main direction (5) having the first predetermined shape (30).

3. Method according to the preceding claim, wherein the microneedle (1) comprises a detection active portion (14) covering at least part of the surface of the pointed tip (3), the active portion (14) comprising an electrically conductive face (15) adapted to be covered with a coating for detecting an analyte.

4. Method according to one of the preceding claims, in which the first predetermined shape (30) has a second principal axis (32) oriented along a maximum dimension of the first predetermined shape (30), the substrate (8) made of monocrystalline silicon having on the first face (9) a direction of [110] orientation of the silicon, the second principal axis (32) forming an angle of zero with the direction of [110] orientation of the silicon modulo 45°.

5. Method according to one of the preceding claims, wherein the first predetermined shape (30) is rectangular or square, and wherein the second predetermined shape (31) is selected from a cross, a superposition of several crosses, and a superposition of a square of a cross.

6. Method according to any of the preceding claims, wherein the etching carried out in step c) comprises deep reactive ion etching.

7. Process according to one of the preceding claims, in which the etching carried out in step d) comprises using a solution comprising at least one compound chosen from potash (KOH) and tetramethylammonium hydroxide (TMAH).

8. Method according to one of the preceding claims, in which the first part (17) of the wall forms a first angle (21) with the first face (9) of the substrate (8), an absolute value of the mean of the first angle (21) being between 90° inclusive and 125.26° exclusive.

9. Method according to one of claims 2 to 8, comprising a step e), subsequent to step d), of forming a layer (23) of electrically insulating material on the first part (17) of the wall and on the second part (19) of the wall.

10. Method according to the preceding claim, comprising a step f), subsequent to step e), of filling the hole (13) with an electrically conductive material.

11. Method according to the preceding claim, comprising a step g), subsequent to step f), of etching a second face (24) of the substrate (8) opposite the first face (9) so as to form or reveal the microneedle (1).

12. Method according to the preceding claim, comprising a step h) of partial etching of the layer (23) of electrically insulating material.

13. Method according to one of the preceding claims, in which the microneedle (1) has an aspect ratio greater than 5, in particular greater than 10, and preferably greater than 15.

14. Method according to one of the preceding claims, in which the first height (25) is greater than 500 µm, preferably greater than 750 µm.
